# EUROPÄISCHE PATENTSCHRIFT

(11) **EP 0 564 532 B1**
(45) Veröffentlichungstag und Bekanntmachung des Hinweises auf die Patenterteilung: **30.06.2004**
(21) Anmeldenummer: 92902118.6
(22) Anmeldetag: 20.12.1991
(51) Int. Cl.: C12N 15/51, A61K 39/29, G01N 33/576

(54) **VON PROTEINEN DES HEPATITIS C-VIRUS ABGELEITETE POLYPEPTIDE UND DEREN VERWENDUNG**
POLYPEPTIDES DERIVED FROM PROTEINS OF THE HEPATITIS C VIRUS
POLYPEPTIDES DERIVES DE PROTEINES DU VIRUS DE L'HEPATITE C

(30) Priorität: 21.12.1990 DE 4041304
(43) Veröffentlichungstag der Anmeldung: 13.10.1993
(73) Patentinhaber: Roche Diagnostics GmbH, 68298 Mannheim (DE)
(72) Erfinder: FUCHS, Klaus, D-8024 Deisenhofen (DE); MOTZ, Manfred, D-8000 München 90 (DE); ROGGENDORF, Michael, D-4300 Essen (DE); SOUTSCHEK, Erwin, D-8137 Berg (DE)
(74) Vertreter: Keller, Günter, Dr.
(86) Internationale Anmeldenummer: PCT/DE1991/001020
(87) Internationale Veröffentlichungsnummer: WO 1992/011370

(56) Entgegenhaltungen:
- EP-A- 0 388 232
- EP-A- 0 464 287
- EP-A- 0 468 657
- Japanese Journal of Experimental Medicine, Band 60, Nr. 4, 1990 (Tokyo, JP) H. Okamoto et al.: "Enzyme-linked immunosorbent assay for antibodies against the capsid protein of hepatitis C virus with a synthetic oligopeptide", Seiten 223-233, siehe Seiten 223-233
- Peptide Chemistry 1990, Proceedings of the 28th Symposium on Peptide Chemistry, Osaka, 25-27 Oktober 1990, E. Munekata et al.: "Epitope-mapping of hepatitis C virus constituting protein", Seiten 211-214, siehe Seiten 211-214

## Beschreibung

Seit etwa 15 Jahren ist bekannt, daß außer der Hepatitis A und der Hepatitis B weitere durch Viren übertragene Hepatiden existieren, die in Unkenntnis des Erregers üblicherweise als Non-A-Non-B-Hepatitis bezeichnet wurden. Mittlerweile wird davon ausgegangen, daß diese Gruppe der Non-A-Non-B-Hepatiden durch wenigstens zwei unterschiedliche Viren hervorgerufen werden, die als Hepatitis C oder E bezeichnet werden.

Bei dem Hepatitis C-Virus handelt es sich um ein einsträngiges bekapseltes RNA-Virus mit einem Durchmesser von etwa 50-60 nm. Das Genom besteht aus etwa 10.000 Nukleotiden, wobei Genregionen unterschieden werden können, die für strukturelle Proteine wie Hülle und Kern kodieren. Derartige Strukturproteine können bezeichnet werden mit C oder Core, E oder Envelope und NS. Darüber hinaus umfaßt das Genom die Gene für nicht-strukturelle Komponenten des Virus wie Enzyme usw. Bisher ist zwar bekannt, daß das Genom verschiedene Proteine umfaßt, die einzelnen viralen Proteine sind jedoch kaum bekannt (Schweizer Medizinische Wochenschrift (1990), Vol. 120, S. 117-124).

In der europäischen Patentanmeldung 88.310922 der Chiron Corporation wurde eine Partialnukleotidsequenz des Hepatitis C-Virus veröffentlicht. Es wurde aber herausgefunden, daß die dort offenbarte Nukleotidsequenz nur für die sogenannten Nicht-Struktur-Proteine kodiert. Derjenige Teil der Nukleotidsequenz, der für die Strukturproteine kodiert, wird in dieser Literaturstelle nicht offenbart.

In der europäischen Patentanmeldung Nr. 89.309261.9 werden weitere DNA-Sequenzen eines non-A-non-B-Hepatitisvirus-Antigens offenbart.

Die europäische Patentanmeldung 90.305421.1 der Chiron Corporation offenbart die DNA- und Aminosäure-Sequenz eines HCV-Virus.

Aus dem Stand der Technik ist die Expression von verschiedenen HCV-Viruspeptiden bekannt, die allerdings immer als sogenannte Fusionspeptide exprimiert werden. Hintergrund der Expression als Fusionsprotein ist, daß das Hepatitis C-Virus an die eukaryontische Proteinbiosynthese angepaßt. ist und daß daher allgemein davon ausgegangen wird, daß derartige Polypeptide in bakteriellen Systemen wie dem E.coli-System nur als sogenannte Fusionsproteine exprimiert werden können. Der anfusionierte Teil stammt daher von anderen Proteinen, wie der β-Galactosidase oder der Superoxid-Dismutase ab. Nachteilig bei derartigen Fusionsproteinen ist jedoch, daß, wenn diese Polypeptide zu Nachweisreaktionen verwendet werden, Kreuzreaktionen mit dem anfusionierten. Proteinteil auftreten können, die zu falsch positiven Ergebnissen führen.

Aufgabe der vorliegenden Erfindung ist es daher, solche von Hepatitis-C-virus abstammenden Polypeptide zur Verfügung zu stellen, die wenigstens eine, aber weniger als 15 Aminosäuren aufweisen, die von Fremdproteinen abstammen, aber dennoch in guter Ausbeute exprimiert werden können.

Unter Fusionsproteinen werden in der vorliegenden Anmeldung solche Polypeptide verstanden, die einen wesentlichen Teil eines Fremdproteins, wie β-Galactosidase oder Superoxid-Dismutase aufweisen.

Anhand der hier offenbarten Verfahren konnten Polypeptide aus den Strukturproteinen C (Core) und ENV (Envelop) kloniert und sequenziert werden. Wesentlich ist, daß diese Polypeptide Teile von Strukturproteinen sind, die in dem Viruspartikel an der Oberfläche oder oberflächennahen Bereichen auftreten. Dadurch kommen Sie mit dem Immunsystem in Kontakt und rufen so eine Immunantwort hervor. Diese Immunantwort ist einerseits wesentlich für den Nachweis einer Infektion, wobei festgestellt wird, ob Antikörper gegen das Virus vorhanden sind und andererseits für die Immunisierung zum Schutz gegen virale Infektionen.

Anhand der offenbarten Aminosäuresequenz können ohne größere Schwierigkeiten verkürzte Polypeptide hergestellt werden, die dieselben oder vergleichbare immunologische Eigenschaften aufweisen wie die hier beschriebenen Polypeptide. Ebenso können die offenbarten Aminosäuresequenzen durch Austausch weniger Aminosäuren so geringfügig verändert, werden, daß die immunologischen Eigenschaften der Polypeptide erhalten bleiben. Im Rahmen der vorliegenden Erfindung werden daher solche Polypeptide bevorzugt, die Teilsequenzen aus den offenbarten Aminosäuresequenzen darstellen, oder sich von den offenbarten Sequenzen durch Austausch weniger Aminosäure unterscheiden. Die ausgetauschten Aminosäuresequenzen sollten jedoch 2 % der gesamten Aminosäuren nicht überschreiten. weiterhin wurden im Rahmen der vorliegenden Erfindung drei Klone zur Verfügung gestellt, die unterschiedliche, definierte Polypeptide aus dem Genom des Hepatitis-C-Virus liefern.

Ausgangsmaterial für die Herstellung der verschiedenen, die Polypeptide von Hepatitis-C-Virus liefernden Klone war das Serum eines virusinfizierten Patienten.

Das Serum wurde nach an sich bekannten Verfahren vorbehandelt und mit Hilfe der "polymerase chain reaction" (PCR) wurden die einzelnen Klone erhalten.

Der inzwischen bei der Deutschen Sammlung für Mikroorganismen (DSM) unter der Nummer 6847 hinterlegte Klon NS-3 enthält die genetische Information für ein Polypeptid von 527 Aminosäuren. An dem N-Terminus des von dem Klon NS-3 produzierten Polypeptids sind einige Aminosäuren vorhanden, die durch die Klonierung entstanden sind und von dem Vektor (pUC) abstammen. Diese wenigen Nonsens-Aminosäuren stellen jedoch keinen Fusionsanteil dar. Das klonierte Polypeptid wurde zwischenzeitlich teilweise sequenziert und weist am C-Terminus folgende Partialsequenz auf:

CC CTC ATG ACC GGC TAT ACC GGC GAC TTC GAC TCG GTG ATA GAC
LEU MET THR GLY TYR THR GLY ASP PHE ASP SER VAL ILE ASP

TGC AAC ACG TGT GTC ACT CAG ACA GTC GAT TTC AGC CTT GAC CCT
CYS ASN THR CYS VAL THR GLN THR VAL ASP PHE SER LEU ASP PRO

ACC TTC ACC ATT GAG ACG ACC ACA CTT CCC CAG GAT GCT GTC TCC
THR PHE THR ILE GLU THR THR THR LEU PRO GLN ASP ALA VAL SER

CGC ACT CAA CGA CGG GGC AGG ACT GGC AGG GGG AAG CCG GGC ATC
ARG THR GLN ARG ARG GLY ARG THR GLY ARG GLY LYS PRO GLY ILE

TAC AGA TTT GTG GCA CCG GGG GAA CGC CCC TCC GGC ATG TTC GAC
TYR ARG PHE VAL ALA PRO GLY GLU ARG PRO SER GLY MET PHE ASP

TCG TCC GTC CTC TGT GAG TGC TAT GAC GCA GGT TGT GCT TGG TAT
SER SER VAL LEU CYS GLU CYS TYR ASP ALA GLY CYS ALA TRP TYR

GAG CTC ACG CCC GCC GAG TGA ATTCAAGCTT
GLU LEU THR PRO ALA GLU STOP

Der Klon NS-4 enthält die genetische Information für ein Polypeptid von 247 Aminosäuren und wurde bei der DSM unter der Nummer 6848 hinterlegt. Das durch den Klon NS-4 herstellbare Polypeptid von Hepatitis-C-Virus enthält ebenfalls am N-Terminus einige Aminosäuren, die von dem Vektor pUC herstammen und auch keinen Fusionsanteil im Sinne eines anfusionierten Fremdproteins darstellen.

Der Klon pIC19 H-N512 wurde bei der DSM unter der Nummer 6849 hinterlegt. Der Klon pIC19H-N512 enthält die Information für ein Polypeptid von Hepatitis-C-Virus, das 392 Aminosäuren aufweist. Auch hier sind am N-Terminus einige wenige Aminosäuren vorhanden, die von dem Vektor pUC herstammen. Dieses HCV-spezifische Peptid weist am N-Terminus folgende Aminosäuresequenz, kodiert von der ebenfalls aufgeführten DNA-Sequenz, auf:

ATC CCC TTT GTG TCC TGC CAG CGC GGG TAT AGG GGG GCC TGG CGA
ILE PRO PHE VAL SER CYS GLN ARG GLY TYR ARG GLY ALA TRP ARG

GGG GAC GGC ATC ATG CAC ACT CGC TGC CAC TGT GGA GCT GAG ATC
GLY ASP GLY ILE MET HIS THR ARG CYS HIS CYS GLY ALA GLU ILE

ACC GGA CAT GTC AAG AAC GGG ACG ATG AGG ATC GTC GGT CCT AAG
THR GLY HIS VAL LYS ASN GLY THR MET ARG ILE VAL GLY PRO LYS

ACC TGC AGG AAC ATG TGG AGT GGG ACC TTC CCC ATT AAT GCC TAC
THR CYS ARG ASN MET TRP SER GLY THR PHE PRO ILE ASN ALA TYR

ACC ACG GGC CCC TGT ACC CCC CTT CCT GCG CCG AAC TAT
THR THR GLY PRO CYS THR PRO LEU PRO ALA PRO ASN TYR

Die im Rahmen der vorliegenden Anmeldung offenbarten Polypeptide weisen den Vorteil auf, daß sie in guter Ausbeute (ca. 5 % des Gesamtproteins) exprimiert werden können.

Überraschenderweise wurde weiterhin festgestellt, daß das erfindungsgemäße, aus dem Strukturprotein (Core) abstammende Polypeptid unter physiologischen Bedingungen löslich ist. Unter physiologischen Bedingungen wird diesbezüglich beispielsweise verstanden, löslich in Tris-Puffer oder Phosphat-Puffer, die etwa 0,5 M NaCl enthalten.

Die Löslichkeit von Polypeptiden spielt dann eine wesentliche Rolle, wenn die Polypeptide eingesetzt werden in sogenannten "Capture"-Tests, bei denen ein Anti-Antikörper an die Festphase gebunden wird, das zu untersuchende Serum mit der Festphase reagieren kann und dann das Antigen in gelöster Form zugegeben wird. Das Antigen weist dabei üblicherweise eine Markierung auf, die den Nachweis von Antikörpern erlaubt, die gegen das Antigen gerichtet sind.

Die erfindungsgemäß hergestellten Polypeptide finden vor allem Verwendung in Testkits, die zum Nachweis von gegen Hepatitis C-Virus gerichtete Antikörper geeignet sind. Unter Testkit wird eine Anordnung verstanden, die als wesentlichen Bestandteil wenigstens ein erfindungsgemäßes Polypeptid aufweist. Üblicherweise ist das Polypeptid an eine feste Phase gebunden, die mit der zu untersuchenden Probe in Kontakt gebracht wird. Bei der zu untersuchenden Probe handelt es sich üblicherweise um Serumproben, jedoch kann auch der Nachweis in anderen Antikörper-enthaltenden biologischen Flüssigkeiten geführt werden.

Nachgewiesen wird der aus erfindungsgemäßem Polypeptid und dagegen gerichtetem Antikörper gebildete Komplex durch eine Anzeigekomponente. Dabei handelt es sich in bevorzugter Weise um einen Antikörper, der gegen den nachzuweisenden Antikörper gerichtet ist. Dieser Anti-Antikörper weist üblicherweise eine Markierung auf, die den Nachweis ermöglicht. Bei den Markierungen kann es sich beispielsweise um eine radioaktive Markierung oder eine Markierung mit einem eine Farbreaktion katalysierenden Enzym handeln.

Im Rahmen der vorliegenden Erfindung werden zwei Ausführungsformen von Testkits besonders bevorzugt. Bei der einen Ausführungsform, die auch als Strip-ELISA bezeichnet wird, handelt es sich um einen Testkit in Form eines Teststreifens. Dieser Teststreifen weist verschiedene als Antigen wirkende Proteine auf. Deren Anordnung erfolgt dabei ähnlich der eines Western-Blots. Die Streifen enthalten wenigstens ein erfindungsgemäßes Polypeptid und zusätzlich ein oder mehrere Kontrollproteine, die zur Intensitätsbeurteilung der Antigen-Antikörperreaktion beitragen.

In bevorzugter Weise werden die Polypeptide bzw. Proteine elektrophoretisch aufgetrennt und dann auf den Teststreifen transferiert. In einer besonders bevorzugten Ausführungsform werden die Polypeptide bzw. Proteine auf einen Nitrocellulosefilter übertragen, der dann in Streifen geschnitten wird.

Die Testdurchführung und Auswertung erfolgt analog zu den an sich bekannten ELISA-Tests und den an sich bekannten Western-Blots.

Zunächst werden auf einem Teststreifen die entsprechenden Proteine bzw. Polypeptide aufgetragen und an die feste Oberfläche gebunden. Diese Teststreifen werden dann in einem Verdünnungspuffer mit der Probe inkubiert. Nach einer ausreichenden Inkubation wird der Teststreifen gewaschen und die Anzeigekomponente zugegeben. Hierbei handelt es sich üblicherweise um einen Anti-Antikörper, an den ein farbgebendes Enzym gekoppelt wurde. Ein Beispiel für ein derartiges eine Farbreaktion katalysierendes Enzym ist Peroxydase. Nach abermaligem Waschen wird Substrat, d.h. eine durch das Enzym umsetzbare Verbindung zugegeben und inkubiert. Anhand der sich bildenden bzw. nicht bildenden Farbreaktion kann dann abgelesen werden, ob Antikörper vorhanden sind.

Bei einer weiteren bevorzugten Ausführungsform der erfindungsgemäßen Testkits handelt es sich um die ELISA-Testkits. Bei diesen Testkits wird wenigstens ein erfindungsgemäßes Polypeptid an die feste Phase von Mikrotiterplatten gebunden. Die einzelnen Testkompartimente der Mikrotiterplatte werden mit der den nachzuweisenden Antikörper enthaltenden Flüssigkeit inkubiert. Nach verschiedenen Waschschritten wird dann die Anzeigekomponente zugegeben und abermals gewaschen. Nach Substratzugabe kann dann anhand der Farbreaktion beurteilt werden, ob Antikörper gegen Hepatitis C-Viren vorhanden sind.

Ein weiteres Anwendungsgebiet der erfindungsgemäßen Polypeptide ist die Herstellung von Impfstoffen. Dabei können die erfindungsgemäßen Polypeptide oder von diesen abstammende kürzere Polypeptide zunächst gentechnologisch in hoher Reinheit hergestellt werden. Die Polypeptide werden dann in die Form von injizierbaren Flüssigkeiten gebracht, wobei es sich entweder um Lösungen oder Suspensionen handeln kann. Dabei können die Polypeptide auch emulgiert werden oder die Polypeptide können in Liposomen eingeschlossen werden. Weitere Bestandteile der Impfstoffe sind beispielsweise Wasser, Salzlösungen, Glucose oder Glycerin. Darüber hinaus enthalten ,die Impfstoffe geringe Mengen an Hilfssubstanzen wie Emulgiermittel, den pH-Wert pufferende Mittel und gegebenenfalls Adjuvantien, die die Immunantwort steigern. Als Adjuvantien können genannt werden Aluminiumhydroxyd, N-Acetyl-muramyl-L-threonyl-D-isoglutamin und ähnliche, an sich bekannte Verbindungen. Die Impfstoffe werden üblicherweise parenteral durch Injektion, in bevorzugter Weise subkutan oder intramuskulär, appliziert. Es hat sich im Rahmen der vorliegenden Erfindung als vorteilhaft herausgestellt, daß die beanspruchten Polypeptide in hoher Ausbeute exprimiert werden können. Üblicherweise werden Proteine, die sich nicht ohne weiteres gentechnologisch darstellen lassen, in der Form von Fusionsproteinen exprimiert. Dabei werden die gewünschten Proteine bzw. Polypeptide an andere, meist bakterielle Proteine, die gut exprimiert werden anfusioniert. Erhalten wird dann ein sogenanntes Fusionsprotein, das einen anfusionierten Proteinanteil und das gewünschte Polypeptid bzw. Protein enthält, wobei beide Teile miteinander zu einem sogenannten Fusionsprotein verschmolzen sind.

Insbesondere für immunologische Nachweisverfahren oder die Herstellung von Impfstoffen ist jedoch die Darstellung der gewünschten Polypeptide als Fusionsprotein nicht erwünscht, da nicht auszuschließen ist, daß auch Antikörper gegen den anfusionierten Proteinteil bestehen, die bei den immunologischen Nachweisverfahren Antikörper gegen das virale Antigen vortäuschen, wo tatsächlich nur Antikörper gegen den anfusionierten Proteinteil existieren.

Auch bei der Immunisierung im Rahmen von Impfstoffen ist es nicht erwünscht, daß das Protein oder Polpeptid gegen das Antikörper erzeugt werden sollen, eine Verunreinigung mit einem anderen Protein bzw. Proteinteil aufweist, da in diesem Fall auch gegen diesen Bestandteil Antikörper gebildet werden. Im Rahmen der vorliegenden Erfindung ist es nun gelungen, die erfindungsgemäßen Polypeptide in reiner Form darzustellen. Die erfindungsgemäßen Polypeptide weisen wenigstens eine aber höchstens 15 Aminosäuren auf, die von anderen Genen herstammen, mit denen das Gen für die erfindungsgemäßen Polypeptide im Rahmen der Klonierung bzw. Expression in Verbindung gebracht wurde. Besonders bevorzugt weisen jedoch die erfindungsgemäßen Polypeptide weniger als 5 Aminosäuren auf, die von auf die Klonierung zurückgehenden Artefakten herstammen.

### Beispiel 1

### Gewinnung der HCV-CORE und ENV-1 kodierenden Sequenzen

Ausgehend von der bekannten Sequenz (Europäische Patentanmeldung 88310922) wurden DNA-Primer synthetisiert, die bei einer c-DNA Synthese als Start in Richtung 5'-Ende des viralen Genoms dienen, sowie ein Primer der am 5' -Ende des c-DNA Primers liegt und der als Hybridisierungsprobe verwendet wird.

Der Primer für die c-DNA Synthese hat folgende Sequenz: 5'-GGGAGTGAAGCAATATACCGGACC Für die Hybridisierung:

5'-CCGATTTTGACCAGGGCTGGGGCCCTATCAGTTAT

5ml Plasma eines chronisch HCV-infizierten Patienten wurde auf ein 20%iges Succrose-Kissen gegeben und für 2h bei 100.000g zentrifugiert. Das Pellet wurde in 300µl Puffer mit 20mM Tris-HCl pH8,0, 200mM NaCl, 10mM EDTA, 2% SDS, 1mg/ml Proteinase K resuspendiert und für 1,5h bei 55°C inkubiert. Nukleinsäuren wurden dann nach einer Phenol/Chloroform-Behandlung mit Ethanol präzipitiert und anschließend wieder in 50 µl Wasser aufgenommen.

Die RNA dieser Lösung wurde nach üblichen Methoden mit dem oben angeführten Primer revers transkribiert und in einen lambda gt11 Phagen eingesetzt. HCV-DNA positive Klone wurden mittels Hybridisierung mit dem Hybridisierung-Primer gefunden. Hierfür war dieser Primer mit Digoxygeninmarkiertem UTP und terminaler Transferase an den Enden markiert worden. Der Nachweis des gebundenen Primers. erfolgte dann mit einem kommerziellen Kit (Boehringer Mannheim).

Hybridisierungs-positive Phagen wurden auf die Größe ihres HCV-Inserts mittels Restriktionsverdau untersucht. Ein Klon mit einem Insert von etwa 1,7kB wurde in einen pUC8-Vektor subkloniert und die Sequenz bestimmt. Ausgehend von kommerziellen pUC-Sequenzierungs-Primern wurde dabei zuerst die Sequenz der Insert-Enden bestimmt, anschließend Primer mit Sequenzen der neu bestimmten 3'-Enden synthetisiert und diese dann für die nächste Sequenzierungsreaktion benutzt. Aus der so bestimmten Sequenz. ergibt sich ein durchgehender Leserahmen, der 169 Nukleotide vom Anfang des Inserts beginnt.

Bei HCV liegen am Anfang des Polyproteins die kodierenden Bereiche des core oder Kapsid-Proteins sowie daran anschließend zwei Bereiche für Membranproteine.

Der Bereich des core-Proteins sowie des unmittelbar nachfolgenden Membran-Proteins wurde für eine Expression in E.coli subkloniert. Dies erfolgte mittels spezifischer DNA-Primer und PCR (polymerase chain reaction). Hierbei wird der für das Protein kodierende Bereich der DNA amplifiziert.

Die Primer-Sequenzen, die zur Amplifizierung des DNA-Bereiches dienten, der anschließend kloniert wurde und zur Expression von core-Protein führte, waren: 5'- gagggatccatc ATG AGC ACA AAT CCT AAA CC für das 5'-Ende 5'- gagaagctta GGA AGC GGG GAT GGT TCA AGc für das 3'-Ende

Die Primer-Sequenzen, die zur Amplifizierung des DNA-Bereiches dienten, der anschließend kloniert wurde und zur Expression von ENV-1-Protein führte, waren: gagggatcc GCT TAC GGA GTG CGC AAC Für das 5'-Ende gagGGATCC GGA CCA GCT TGA GCT ACT AC Für das 3'-Ende

Klein geschriebene Nukleotide sind nicht HCV-spezifisch, sie wurden in den Primern verwendet, um Restriktionsschnittstellen (GGATCC -BamHI, AAGCTT -HindIII, TCATGA - BspHII) für die anschließende Klonierung zur Verfügung zu haben.

Die aus der PCR erhaltenen, amplifizierten DNA-Fragmente (463 Nukleotide für ENV-1, 579 Nukleotide für core) wurden mit BamHI, (ENV-1) bzw. BamHI und HindIII (core) geschnitten und in pUC8 inseriert. "

Ein E.coli Klon mit dem Plasmid pUC8-ENV-1 mit dem kodierenden Bereich des ENV-1 exprimiert nach IPTG-Induktion ein Protein mit der Größe von etwa 25kDal in einer Menge, die eine effiziente Aufreinigung nach konventionellen Methoden (Molekularsieb-, Ionenaustauscher-Chromatographie) erlaubt. pUCB-C1 mit dem core-kodierenden Bereich wird in geringerer Menge produziert; ein Umsetzen des Inserts in andere Expressionsvektoren (pDS, pKK233-2) bringt keine wesentliche Verbesserung. Aber nach Verkürzen des Inserts vom 3'-Ende her erfolgt eine sehr gute Expression in den E. coli-Zellen; das Expressionsprodukt mit ca. 15kDal entspricht der core-Sequenz von Aminosäure 1 bis 124. Dieses Antigen läßt sich nün ebenfalls sehr effizient mit konventionellen Methoden reinigen.

### Beispiel 2

Expression und Immunreaktion der HCV-Proteine "core" und "env1".

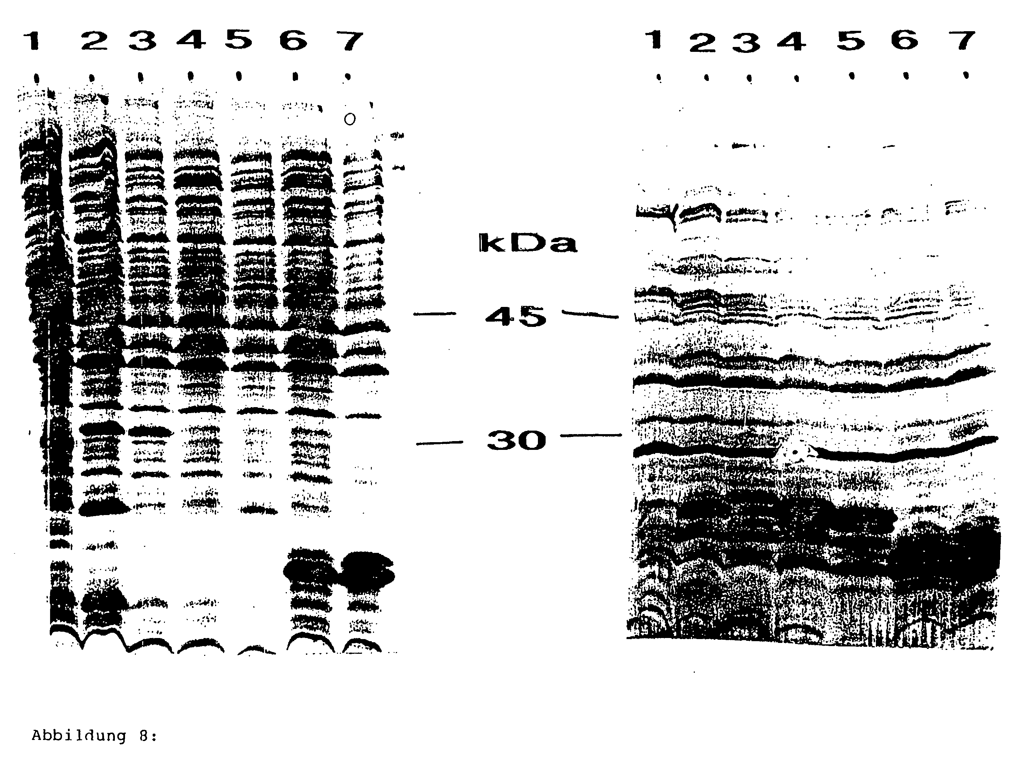

Escherichia coli Zellen mit den entsprechenden Expressionsplasmiden wurden in Flüssig-Kultur angezogen und bei Erreichen einer optischen Dichte (600nm) von 0,7 mit 2 mM IPTG (Isopropyl-β-thiogalaktosid) für drei Stunden induziert. Das Zellpellet aus 1,5 ml Kultur wurde anschließend in 300 µl 2% SDS, 5% Mercaptoethanol, 20 mM Tris-HCl pH 7,5, 2% Bromphenolblau, 30% Succrose resuspendiert und bei 100°C für 5 min inkubiert. Eine Auftrennung der Proteine erfolgte in einem 17,5% SDS-Polyacrylamid-Gel (DADT-quervernetzt). Die Gele sind in Abb. 8 gezeigt.

Das linke Bild zeigt dieses Gel nach Anfärbung der Proteine durch Coomassie-Blau, das Rechte nach einer Immun-Färbung. Hierfür wurden die aufgetrennten Proteine durch "Western Blotting" auf eine Nitrocellulose-Membran transferiert. Die Membran wurde anschließend mit einem HCV-positiven Serum in einer Verdünnung von 1:100 mit TTBS (146 g NaCl, 100 ml 1M Tris-HCl pH 7,5, 0,5 ml Tween-20) für vier Stunden inkubiert, und ungebundene Antikörper durch dreimaliges, 5-minütiges Waschen mit TTBS entfernt. Gebundene Antikörper wurden durch 1-stündige Inkubation mit einem Peroxidasekonjugierten, anti-human-IgG Kaninchen-Antikörper, Waschen mit TTBS und Färbung mit Diaminobenzidin und Peroxid nachgewiesen. Schwächere Banden, die in jeder Spur auftreten, resultieren von anti-E.coli-Antikörpern des verwendeten HCV-Serums.

Zwischen den beiden Abbildungen sind als Molekulargewichtsstandards die Laufweiten von Proteinen mit der Größe von 45 bzw. 30 kDal angegeben.

Die einzelnen Spuren des Geles wurden beladen mit:
- Spur 1:: pUC8, Kontrolle
- Spur 2:: pDS-ENV-1, Expression des HCV-ENV-1 mit einer Größe von ca. 25 kDal.
- Spur 3:: pUC8-C1. Expression des core, Aminosäuren 1-190, Größe ca. 26 kDal, bedingt durch Klonierungsschritte sind noch 20 Aminosäuren anfusioniert.
- Spur 4:: pDS-C1, wie Spur 3, das Produkt ist jedoch kleiner, da weniger Fremdaminosäuren anfusioniert sind.
- Spur 5:: pKK-C1, wie Spur 3 und 4, das Produkt besteht hier nur aus HCV-spezifischen Aminosäuren, Sequenz ist in den Abbildungen angegeben.
- Spur 6:: pDS-C1Cla, verkürztes core-Produkt, von Aminosäure 1 bis 124, am N-Terminus sind noch 2, am C-Terminus eine Fremdaminosäure anfusioniert.
- Spur 7:: pKK-C1-Cla, wie Spur 6, jedoch mit authentischem HCV-N-Terminus, einer Fremdaminosäure am C-Terminus, Sequenz ist in den Abbildungen angegeben.

Das ENV-1 Produkt. wird in relativ hohem Maße exprimiert, reagiert aber mit dem hier getesteten Serum eines chronisch Infizierten nur sehr schwach im Immunoblot. Die core Produkte reagieren alle sehr stark, eine sehr verbesserte Expressions-Ausbeute läßt sich durch Verkürzung des Leserahmen vom C-Terminus her bis zu Aminosäure 124 erreichen.

### Beispiel 3

### Austestung der Reaktivität von core und ENV-1

Für die Bestimmung der Reaktivität von Antikörpern in Patienten-Seren mit diesen Proteinen wurden die beiden gereinigten Antigene nebeneinander auf 17,5% SDS-Polyacryamid-Gelen aufgetrennt und auf Nitrocellulose transferiert. Die Nitrocellulose-Membran wurde nun in schmale Streifen geschnitten, so daß jeweils beide Spuren mit ENV-1 und core sich auf einem Streifen befinden. Die Streifen wurden nun mit verschiedenen Patienten-Seren inkubiert und spezifische Antikörper mittels Immun-Färbung nachgewiesen.

### 1. Test:

22 Seren von Patienten mit erhöhten Transaminasen-Werten, alle HCV-ELISA mit Nicht-Struktur-Proteinen negativ:
1 Serum reaktiv mit core

### 2. Test:

9 Seren von Patienten mit erhöhten Transaminasen-Werten, HCV-ELISA mit Nicht-Struktur-Proteinen grenzwertig
2 Seren reaktiv mit core

### 3. Test:

4 Seren von Hepatitis-Patienten mit Autoimmunreaktivität, alle HCV-ELISA mit Nicht-Struktur-Proteinen positiv
3 Seren reaktiv mit core.

Das Beispiel zeigt, daß Testkits mit den erfindungsgemäßen Polypeptiden sowohl sensitiver (Test 1 und 2) wie auch spezifischer (Test 3) sind, als Testkits bei denen Nicht-Struktur-Proteine eingesetzt werden.

### Beispiel 4

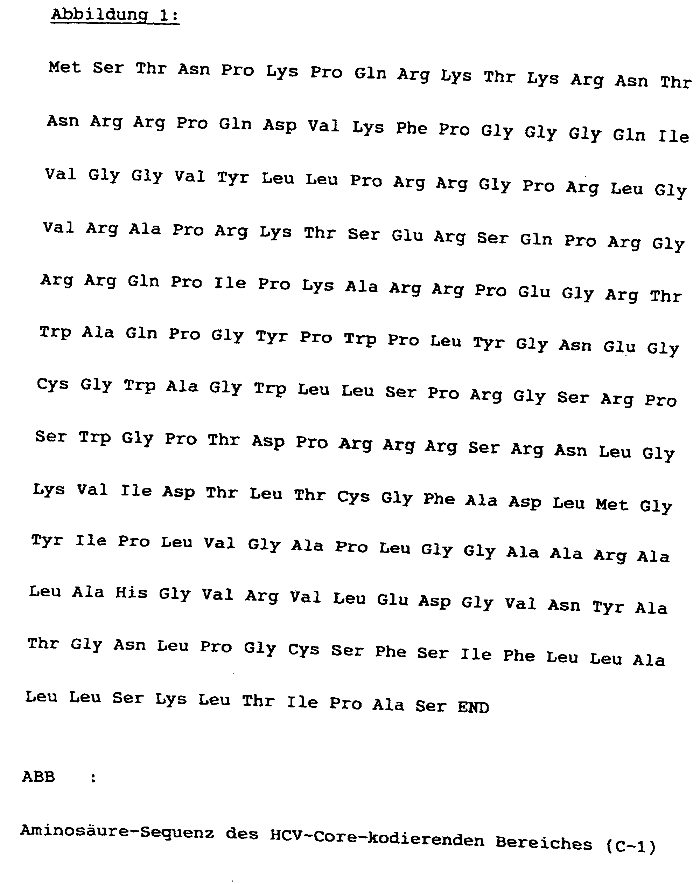

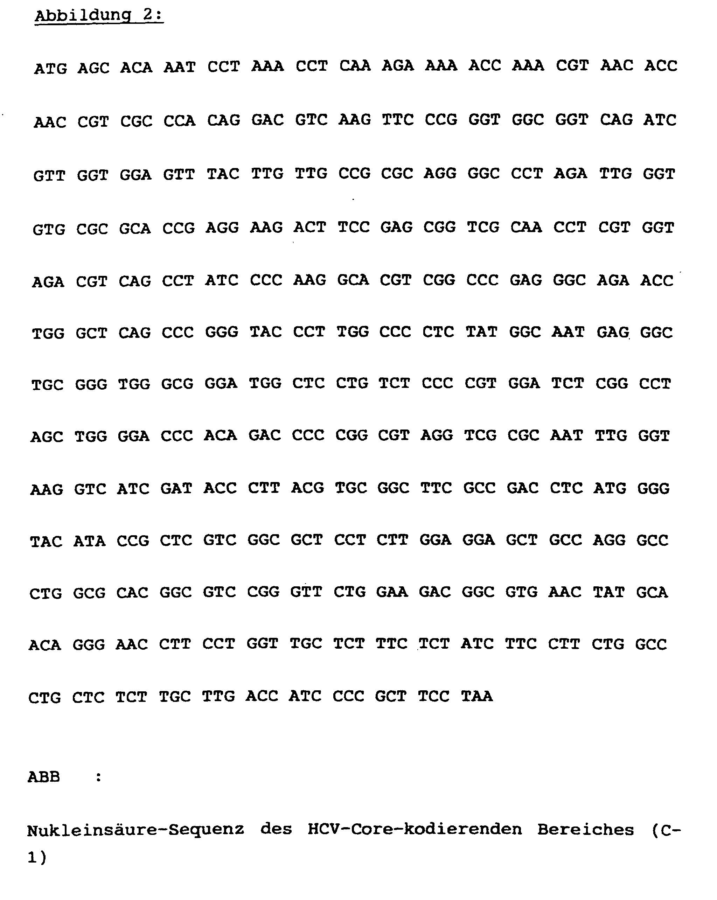

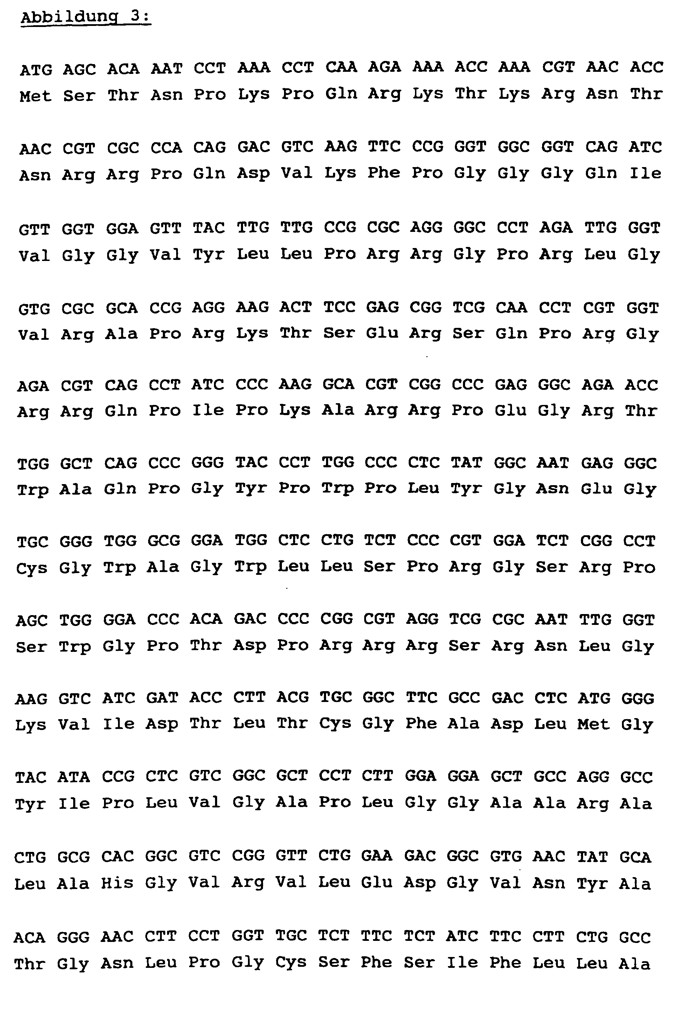

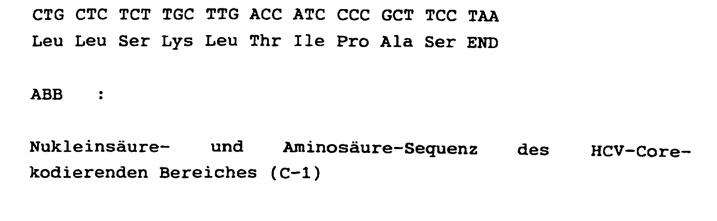

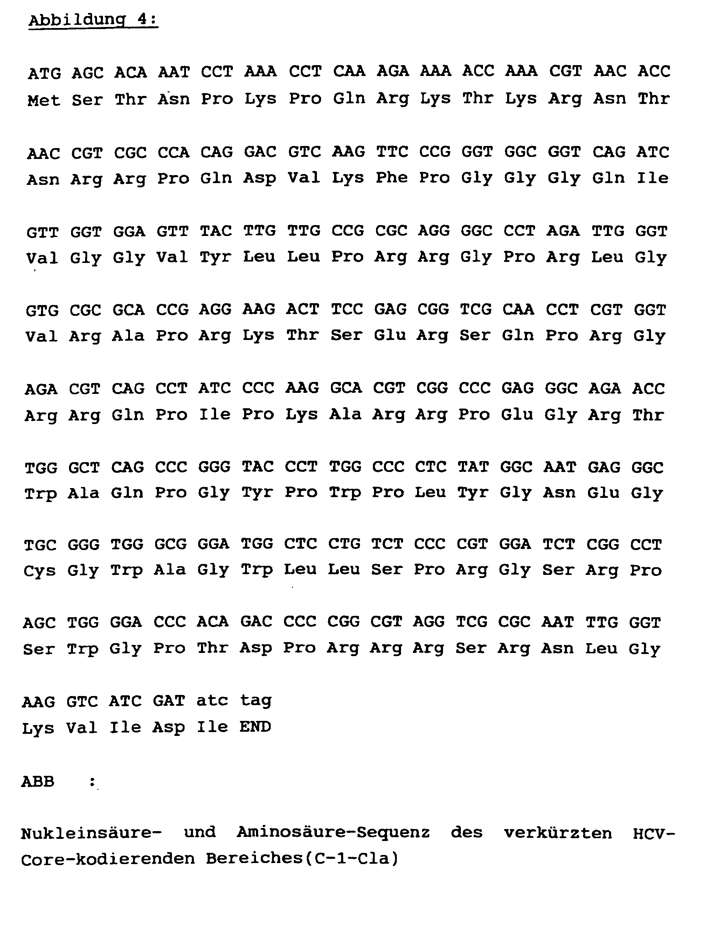

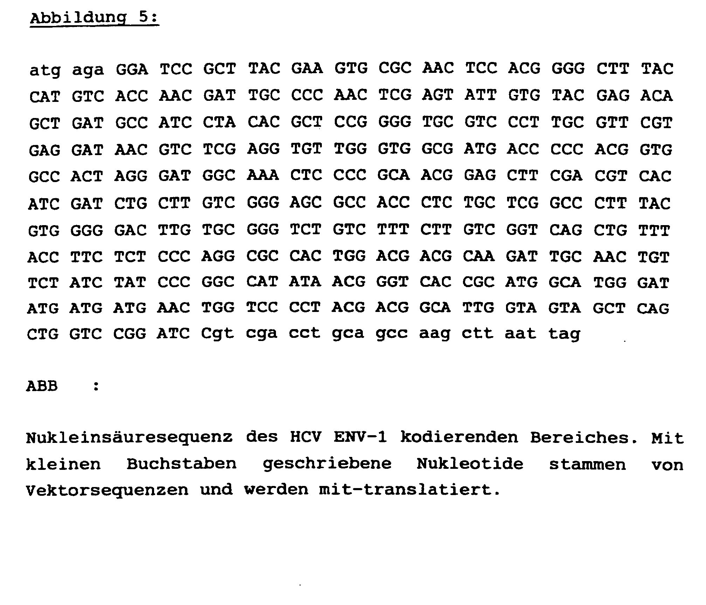

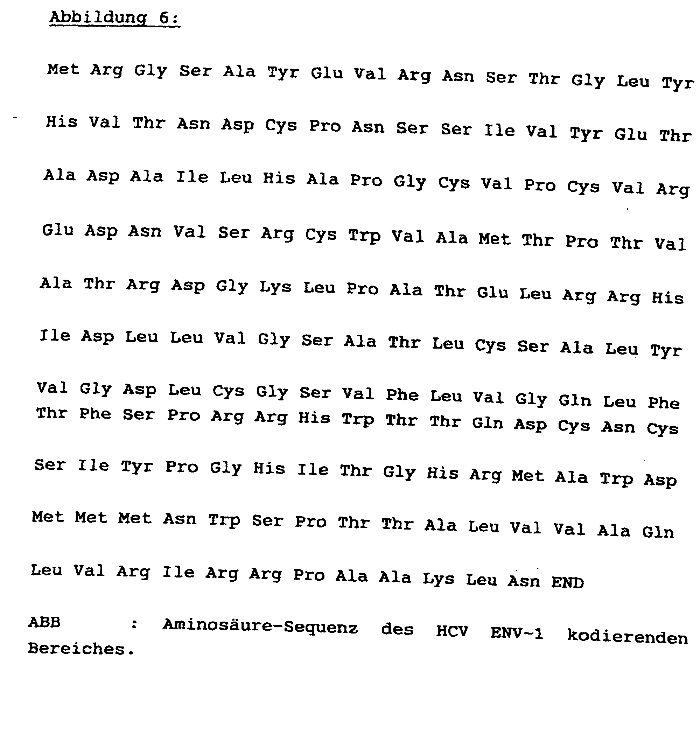

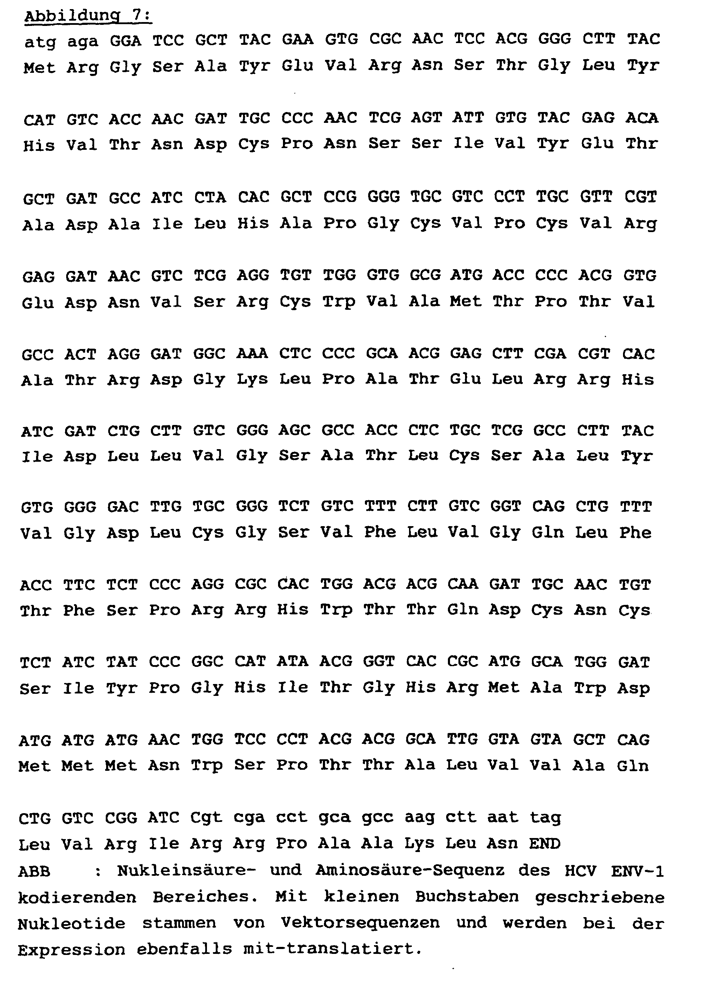

Das in Abb. 4 gezeigte Polypeptid (Core-Antigen) wurde weiter am C-terminalen Ende verkürzt. Dies erfolgte mittels PCR aus dem Core-kodierendem Plasmid (Beispiel 1), dem 5'-PCR-Core-Primer aus Beispiel 1, sowie einem 3'-Primer mit folgender Sequenz:

c gga agc tta CCT ACG CCG GGG GTC TGT GGG.

Dieser Primer hybridisiert auf dem Core-kodierenden Plasmid bis zu einer Nukleinsäure-Abfolge, die der Aminosäure 115 des Core-Peptids entspricht.. Nachfolgend ist ein Codon für den Translationsstop sowie eine HindIII-Stelle für die Klonierung in pUC8/pKK233-2, analog zu Beispiel 1.

Das Polypeptid weist die in Abbildung 4 gezeigte Aminosäuresequenz auf, die allerdings im Unterschied zur Abbildung 4 nach den drei Arginin-Resten in der vorletzten Zeile der Abbildung 4 endet.

Dieses Polypeptid läßt sich besonders gut exprimieren und reinigen.

Eine Reinigung dieses Polypeptids kann dadurch erfolgen, daß man zunächst die Bildung von sogenannten inclusion bodies abwartet und dann die Zellen lysiert. Das Pellet wird in 8M Harnstoff aufgenommen und auf eine DEAE-Säule aufgebracht (8M Harnstoff; pH 8,8). Die Elution von der DEAE-Säule erfolgt mit einem Salzgradienten, wobei die positiven Fraktionen über eine Q-Sepharosesäule (pH 8,0) geleitet werden. Die aktiven Fraktionen befinden sich dabei im Durchlauf, der weiterhin über eine S-Sepharosesäule (pH 7,7) geleitet wird. Eine Elution des Polypeptids erfolgt dann bei einer Salzkonzentration von etwa 0,4 M NaCl. Das erhaltene Polypeptid kann dann gegen Tris-Puffer dialysiert werden, der 0,5 M NaCl enthält.

### Beispiel 5

Der bei der DSM unter der Nummer 6849 hinterlegte Klon pIC19 H-N512 wurde folgendermaßen erhalten. Wie oben beschrieben, wurde das Serum eines Hepatitis-C-Virus infizierten Patienten behandelt und die virale RNA wurde mit Hilfe der PCR-Methode amplifiziert. Zunächst wurde dann ein erster Vor-Klon hergestellt, der ein Insert von etwa 617 Nukleotiden aufwies. Zur Synthese wurden dabei die folgenden Primer verwendet: 
5'-Primer:
CTGCCTGGGATCCCCTTTGTGTCC
 und

3'-Primer:
GGAAAGCTTAAGCGGATAGCTGGCTAGCCGAGGAG.

Kloniert wurde dabei mit den Restriktionsenzymen BamHI/HindIII. Das 3'-Ende dieses Klons enthält vor der HindIII-Schnittstelle eine NheI-Schnittstelle (GCT AGC).

In analoger weise wurde ein weiterer Vor-Klon synthetisiert, der ein Insert von etwa 615 Nukleotiden enthielt. Verwendet wurden hier die folgenden Primer:

5'-Primer:
GAGGGATCCAGGGGATCACCCCCCTCTGTGGCC;

3'-Primer:
GAGAAGCTTGAATTCTATGTGACTTTCTTCTGCCTTTGGCAAG.

Das 5'-Ende dieses Klons (kloniert mit BamHI/HindIII) ist mit dem 1. Vorklon überlappend und enthält ebenfalls die NheI-Schnittstelle.

Die Inserts der beiden Vor-Klone wurden durch Umklonierung unter Verwendung der NheI-Schnittstelle zusammengebaut und dabei wurde der oben erwähnte Klon pIC19H-N512 erhalten. Das Hepatitis C-spezifische DNA-Insert wurde ansequenziert und es wurde am N-Terminus die folgende Partialsequenz (DNA und Aminosäure) ermittelt:

ATC CCC TTT GTG TCC TGC CAG CGC GGG TAT AGG GGG GCC TGG CGA
ILE PRO PHE VAL SER CYS GLN ARG GLY TYR ARG GLY ALA TRP ARG

GGG GAC GGC ATC ATG CAC ACT CGC TGC CAC TGT GGA GCT GAG ATC
GLY ASP GLY ILE MET HIS THR ARG CYS HIS CYS GLY ALA GLU ILE

ACC GGA CAT GTC AAG AAC GGG ACG ATG AGG ATC GTC GGT CCT AAG
THR GLY HIS VAL LYS ASN GLY THR MET ARG ILE VAL GLY PRO LYS

ACC TGC AGG AAC ATG TGG AGT GGG ACC TTC CCC ATT AAT GCC TAC
THR CYS ARG ASN MET TRP SER GLY THR PHE PRO ILE ASN ALA TYR

ACC ACG GGC CCC TGT ACC CCC CTT CCT GCG CCG AAC TAT
THR THR GLY PRO CYS THR PRO LEU PRO ALA PRO ASN TYR

### Beispiel 6

Der Klon NS-3, der für ein virales Polypeptid von 527 Aminosäuren codiert, wurde bei der DSM unter der Nummer 6847 hinterlegt. Am N-Terminus weist das Polypeptid noch einige Aminosäuren auf, die von dem Klonierungsvektor pUC abstammen, aber sogenannte Nonsens-Aminosäuren darstellen.

In zu Beispiel 5 analoger Weise wurden auch hier zwei sich überlappende Vor-Klone hergestellt, wobei dann die klonierten Inserts miteinander kombiniert wurden. Zu Herstellung des einen Vor-Klons, der ein Insert von 815 Nukleotiden aufwies, wurden die folgenden Primer verwendet:

5'-Primer:
AAGGATCCGGCCGGGAGATACTGCTCGGG;

3'-Primer:
GGCAAGCTTGAATTCAGATGTTAGGATCGATCCCATGAG.

Kloniert wurde hier mit den Restriktionsendonukleasen BamHI/HindIII. Der zweite Vor-Klon wies ein Insert von 791 Nukleotiden auf. Dieser Klon wurde durch Verwendung der folgenden Primer erhalten: 
5'-Primer:
GGAGGATCCGCTCATGGGATCGATCCTAAC
 und

3'-Primer:
GGAAGCTTGAATTCACTCGGCGGGCGTGAGCTCATACCAAG.

Kloniert wurde auch hier mit den Restriktionsendonuklease-Schnittstellen BamHI/HindIII. Zusammengefügt wurden die beiden Klone über eine singuläre ClaI Schnittstelle (ATC GAT), die bei dem ersten Vorklon am 3'-Ende und beim zweiten Vorklon am 5'-Ende lokalisiert ist. Von diesem Klon wurde eine Partialsequenz ermittelt, ausgehend vom C-Terminus des Virus-spezifischen Polypeptids, wobei folgende DNA und Aminosäuresequenz ermittelt wurde:

CC CTC ATG ACC GGC TAT ACC GGC GAC TTC GAC TCG GTG ATA GAC
LEU MET THR GLY TYR THR GLY ASP PHE ASP SER VAL ILE ASP

TGC AAC ACG TGT GTC ACT CAG ACA GTC GAT TTC AGC CTT GAC CCT
CYS ASN THR CYS VAL THR GLN THR VAL ASP PHE SER LEU ASP PRO

ACC TTC ACC ATT GAG ACG ACC ACA CTT CCC CAG GAT GCT GTC TCC
THR PHE THR ILE GLU THR THR THR LEU PRO GLN ASP ALA VAL SER

CGC ACT CAA CGA CGG GGC AGG ACT GGC AGG GGG AAG CCG GGC ATC
ARG THR GLN ARG ARG GLY ARG THR GLY ARG GLY LYS PRO GLY ILE

TAC AGA TTT GTG GCA CCG GGG GAA CGC CCC TCC GGC ATG TTC GAC
TYR ARG PHE VAL ALA PRO GLY GLU ARG PRO SER GLY MET PHE ASP

TCG TCC GTC CTC TGT GAG TGC TAT GAC GCA GGT TGT GCT TGG TAT
SER SER VAL LEU CYS GLU CYS TYR ASP ALA GLY CYS ALA TRP TYR

GAG CTC ACG CCC GCC GAG TGA ATTCAAGCTT
GLU LEU THR PRO ALA GLU STOP

### Beispiel 7

Der Klon NS-4, der mittlerweile bei der DSM unter der Nummer 6848 hinterlegt wurde, codiert für ein, Polypeptid von 247 Aminosäuren, das von einem viralen Protein abstammt. Auch dieses Polypeptid weist am N-Terminus einige Aminosäuren auf, die von dem Vektor pUC abstammen und für sogenannte Nonsens-Aminosäuren codieren. Hergestellt wurde dieser Klon nach den vorbeschriebenen Methoden. Verwendet wurden dabei folgende Primer:

5'-Primer:
GGAGGATCCCCCACCCTCCATGGGCCAACACCCC;

3'-Primer:
GGGAAGCTTGAATTCAAAGAGCTCCCGCCACGCCCGC.

Kloniert wurde dieses PCR-Fragment mittels BamHI (5'-Ende) und HindIII (3'-Ende) in pUC8.

### Beschreibung der Abbildungen:

### Abbildung 1:

Es wird die Aminosäuresequenz des HCV-Core-kodierenden Bereiches (C-1) dargestellt.

### Abbildung 2:

Es wird die Nukleinsäuresequenz des HCV-Core-kodierenden Bereiches (C-1) dargestellt.

### Abbildung 3:

Es wird die Korrelation zwischen Nukleinsäure- und Aminosäuresequenz des HCV-Core-kodierenden Bereiches (C-1) dargestellt.

Abbildung 4: Es wird die Nukleinsäure- und Aminosäuresequenz des verkürzten HCV-Core-kodierenden Bereiches dargestellt, das die Aminosäuresequenz 1-123 aufweist und einem Expressionsprodukt mit ca. 15 kD entspricht.

### Abbildung 5:

Es wird die Nukleinsäüresequenz des ENV (Envelop)-ködierenden Bereiches des Virus Hepatitis C (HCV) dargestellt, wobei die mit kleinen Buchstaben geschriebenen Nukleotide von den Sequenzen des Vektors stammen und mittranslatiert werden.

### Abbildung 6:

Es wird die Aminosäuresequenz des ENV (Envelop) Polypeptids des Hepatitis C-Virus dargestellt.

### Abbildung 7:

Es wird die Korrelation zwischen Nukleinsäure- und Aminosäuresequenz des ENV (Envelop) Polypeptidbereiches von Hepatitis C-Virus dargestellt. Die mit kleinen Buchstaben geschriebenen Nukleotidsequenzen stammen von den Sequenzen des Vektors und werden bei der Expression mit-translatiert.

### Abbildung 8:

Es werden die in Beispiel 2 beschriebenen Gele dargestellt.

## Patentansprüche

1. Gentechnologisch hergestelltes Polypeptid aus dem Core(C)-Protein des Hepatitis C-Virus, **dadurch gekennzeichnet, daß** das Core(C)-Protein die Aminosäuresequenz 
Met Ser Thr Asn Pro Lys Pro Gln Arg Lys Thr Lys Arg Asn Thr
Asn Arg Arg Pro Gln Asp Val Lys Phe Pro Gly Gly Gly Gln Ile
Val Gly Gly Val Tyr Leu Leu Pro Arg Arg Gly Pro Arg Leu Gly
Val Arg Ala Pro Arg Lys Thr Ser Glu Arg Ser Gln Pro Arg Gly
Arg Arg Gln Pro Ile Pro Lys Ala Arg Arg Pro Glu Gly Arg Thr
Trp Ala Gln Pro Gly Tyr Pro Trp Pro Leu Tyr Gly Asn Glu Gly
Cys Gly Trp Ala Gly Trp Leu Leu Ser Pro Arg Gly Ser Arg Pro
Ser Trp Gly Pro Thr Asp Pro Arg Arg Arg Ser Arg Asn Leu Gly
Lys Val Ile Asp
 oder Teilsequenzen davon aufweist, wobei die Teilsequenzen am C-terminalen Ende bis Aminosäure 115 verkürzt sein können und das gentechnologisch hergestellte Polypeptid wenigstens eine, aber weniger als 15 Aminosäuren aufweist, die von anfusionierten Fremdproteinen abstammen oder von dem Klonierungsvektor herstammen.

2. Testkit zum Nachweis von gegen Hepatitis C-Virus Antigen gerichteten Antikörpern in einer Probe, **dadurch gekennzeichnet, daß** das Testkit ein Polypeptid nach Anspruch 1 enthält.

3. Testkit nach Anspruch 2, **dadurch gekennzeichnet, daß** das Polypeptid an eine feste Phase gebunden ist.

4. Testkit nach einem der Ansprüche 2 oder 3, **dadurch gekennzeichnet, daß** es darüber hinaus eine Anzeigekomponente für den aus Polypeptid und nachzuweisendem Antikörper gebildeten Komplex aufweist.

5. Testkit nach einem der Ansprüche 2 bis 4, **dadurch gekennzeichnet, daß** es sich um einen Teststreifen handelt, auf den wenigstens 1 Polypeptid gemäß Anspruch 1 aufgebracht wurde und der weiterhin wenigstens 1 Kontrollprotein zur Intensitätsbeurteilung der Antigen-Antikörper-Reaktion aufweist.

6. Testkit nach einem der Ansprüche 2 bis 4, **dadurch gekennzeichnet, daß** es sich um einen ELISA-Testkit handelt.

7. Nukleotid-Sequenz, die für ein HCV-Core-Polypeptid kodiert, **dadurch gekennzeichnet, daß** sie folgende DNA-Sequenz aufweist:

ATG AGC ACA AAT CCT AAA CCT CAA AGA AAA ACC AAA CGT AAC ACC
AAC CGT CGC CCA CAG GAC GTC AAG TTC CCG GGT GGC GGT CAG ATC
GTT GGT GGA GTT TAC TTG TTG CCG CGC AGG GGC CCT AGA TTG GGT
GTG CGC GCA CCG AGG AAG ACT TCC GAG CGG TCG CAA CCT CGT GGT
AGA CGT CAG CCT ATC CCC AAG GCA CGT CGG CCC GAG GGC AGA ACC
TGG GCT CAG CCC GGG TAC CCT TGG CCC CTC TAT GGC AAT GAG GGC
TGC GGG TGG GCG GGA TGG CTC CTG TCT CCC CGT GGA TCT CGG CCT
AGC TGG GGA CCC ACA GAC CCC CGG CGT AGG TCG CGC AAT TTG GGT
AAG GTC ATC GAT ACC CTT ACG TGC GGC TTC GCC GAC CTC ATG GGG
TAC ATA CCG CTC GTC GGC GCT CCT CTT GGA GGA GCT GCC AGG GCC
CTG GCG CAC GGC GTC CGG GTT CTG GAA GAC GGC GTG AAC TAT GCA
ACA GGG AAC CTT CCT GGT TGC TCT TTC TCT ATC TTC CTT CTG GCC
CTG CTC TCT TGC TTG ACC ATC CCC GCT TCC TAA

8. Verfahren zum Nachweis von gegen Hepatitis C-Virus-Antigen gerichteten Antikörpern in einer Untersuchungsprobe, **dadurch gekennzeichnet, daß** bei dem Verfahren polypeptide nach Anspruch 1 eingesetzt werden.

## Claims

1. A polypeptide, prepared by genetic engineering from the core (C) protein of the hepatitis C virus, **characterized in that** the core (C) protein has the amino acid sequence 
Met Ser Thr Asn Pro Lys Pro Gln Arg Lys Thr Lys Arg Asn Thr
Asn Arg Arg Pro Gln Asp Val Lys Phe Pro Gly Gly Gly Gln Ile
Val Gly Gly Val Tyr Leu Leu Pro Arg Arg Gly Pro Arg Leu Gly
Val Arg Ala Pro Arg Lys Thr Ser Glu Arg Ser Gln Pro Arg Gly
Arg Arg Gln Pro Ile Pro Lys Ala Arg Arg Pro Glu Gly Arg Thr
Trp Ala Gln Pro Gly Tyr Pro Trp Pro Leu Tyr Gly Asn Glu Gly
Cys Gly Trp Ala Gly Trp Leu Leu Ser Pro Arg Gly Ser Arg Pro
Ser Trp Gly Pro Thr Asp Pro Arg Arg Arg Ser Arg Asn Leu Gly
Lys Val Ile Asp
 or partial sequences thereof, wherein the partial sequences can be shortened at the C terminal end up to amino acid 115, and the polypeptide prepared by genetic engineering has at least one but less than 15 amino acids which originate from foreign proteins that are fused on or from the cloning vector.

2. A test kit for the detection of antibodies in a sample directed against hepatitis C virus antigen, **characterized in that** the test kit contains a polypeptide of claim 1.

3. The test kit of claim 2, **characterized in that** the polypeptide is bound to a solid phase.

4. The test kit of one of claims 2 or 3, **characterized in that**, in addition, it has an indicator component for the complex formed from polypeptide and the antibody that is to be detected.

5. The test kit of one of claims 2 to 4, **characterized in that** it is a test strip, on which at least one polypeptide of claim 1 has been applied and which furthermore has at least one control protein for evaluating the intensity of the antibody-antigen reaction.

6. The test kit of one of claims 2 to 4, **characterized in that** it is an ELISA test kit.

7. A nucleotide sequence, which codes for an HCV core polypeptide, **characterized in that** it has the following DNA sequence:

ATG AGC ACA AAT CCT AAA CCT CAA AGA AAA ACC AAA CGT AAC ACC
AAC CGT CGC CCA CAG GAC GTC AAG TTC CCG GGT GGC GGT CAG ATC
GTT GGT GGA GTT TAC TTG TTG CCG CGC AGG GGC CCT AGA TTG GGT
GTG CGC GCA CCG AGG AAG ACT TCC GAG CGG TCG CAA CCT CGT GGT
AGA CGT CAG CCT ATC CCC AAG GCA CGT CGG CCC GAG GGC AGA ACC
TGG GCT CAG CCC GGG TAC CCT TGG CCC CTC TAT GGC AAT GAG GGC
TGC GGG TGG GCG GGA TGG CTC CTG TCT CCC CGT GGA TCT CGG CCT
AGC TGG GGA CCC ACA GAC CCC CGG CGT AGG TCG CGC AAT TTG GGT
AAG GTC ATC GAT ACC CTT ACG TGC GGC TTC GCC GAC CTC ATG GGG
TAC ATA CCG CTC GTC GGC GCT CCT CTT GGA GGA GCT GCC AGG GCC
CTG GCG CAC GGC GTC CGG GTT CTG GAA GAC GGC GTG AAC TAT GCA
ACA GGG AAC CTT CCT GGT TGC TCT TTC TCT ATC TTC CTT CTG GCC
CTG CTC TCT TGC TTG ACC ATC CCC GCT TCC TAA

8. A method for the detection of antibodies directed against hepatitis C virus antigen in an analysis sample, **characterized in that** polypeptides of claim 1 are used in the method.

## Revendications

1. Polypeptide préparé par ingénierie génétique à partir de la protéine coeur (C) du virus de l'hépatite C, **caractérisé en ce que** la protéine coeur (C) présente la séquence d'acides aminés : 
Met Ser Thr Asn Pro Lys Pro Gln Arg Lys Thr Lys Arg Asn Thr
Asn Arg Arg Pro Gln Asp Val Lys Phe Pro Gly Gly Gly Gln Ile
Val Gly Gly Val Tyr Leu Leu Pro Arg Arg Gly Pro Arg Leu Gly
Val Arg Ala Pro Arg Lys Thr Ser Glu Arg Ser Gln Pro Arg Gly
Arg Arg Gln Pro Ile Pro Lys Ala Arg Arg Pro Glu Gly Arg Thr
Trp Ala Gln Pro Gly Tyr Pro Trp Pro Leu Tyr Gly Asn Glu Gly
Cys Gly Trp Ala Gly Trp Leu Leu Ser Pro Arg Gly Ser Arg Pro
Ser Trp Gly Pro Thr Asp Pro Arg Arg Arg Ser Arg Asn Leu Gly
Lys Val Ile Asp
 ou des séquences partielles de celle-ci, les séquences partielles pouvant, à l'extrémité terminale C, être raccourcies jusqu'à l'acide aminé 115 et le polypeptide préparé par ingénierie génétique présentant au moins un acide aminé, mais moins de 15 acides aminés, qui proviennent de protéines étrangères fusionnées ou sont originaires du vecteur de clonage.

2. Trousse d'essai pour déceler dans un échantillon des anticorps dirigés contre l'antigène du virus de l'hépatite C, caractérisée en ce la trousse d'essai contient un polypeptide suivant la revendication 1.

3. Trousse d'essai suivant la revendication 2, **caractérisée en ce que** le polypeptide est fixé sur une phase solide.

4. Trousse d'essai suivant l'une des revendications 2 et 3, **caractérisée en ce qu'**elle présente, en outre, un composant indicateur pour le complexe formé du polypeptide et de l'anticorps à déceler.

5. Trousse d'essai suivant l'une des revendications 2 à 4, **caractérisée en ce qu'**il s'agit d'un ruban d'essai sur lequel au moins un polypeptide suivant la revendication 1 a été appliqué et qui présente, en outre, au moins une protéine témoin pour l'évaluation d'intensité de la réaction antigène-anticorps.

6. Trousse d'essai suivant l'une des revendications 2 à 4, **caractérisée en ce qu'**il s'agit d'une trousse d'essai ELISA.

7. Séquence nucléotidique, qui code pour un polypeptide coeur de HCV, **caractérisée en ce qu'**elle présente une séquence d'ADN suivante :

ATG AGC ACA AAT CCT AAA CCT CAA AGA AAA ACC AAA CGT AAC ACC
AAC CGT CGC CCA CAG GAC GTC AAG TTC CCG GGT GGC GGT CAG ATC
GTT GGT GGA GTT TAC TTG TTG CCG CGC AGG GGC CCT AGA TTG GGT
GTG CGC GCA CCG AGG AAG ACT TCC GAG CGG TCG CAA CCT CGT GGT
AGA CGT CAG CCT ATC CCC AAG GCA CGT CGG CCC GAG GGC AGA ACC
TGG GCT CAG CCC GGG TAC CCT TGG CCC CTC TAT GGC AAT GAG GGC
TGC GGG TGG GCG GGA TGG CTC CTG TCT CCC CGT GGA TCT CGG CCT
AGC TGG GGA CCC ACA GAC CCC CGG CGT AGG TCG CGC AAT TTG GGT
AAG GTC ATC GAT ACC CTT ACG TGC GGC TTC GCC GAC CTC ATG GGG
TAC ATA CCG CTC GTC GGC GCT CCT CTT GGA GGA GCT GCC AGG GCC
CTG GCG CAC GGC GTC CGG GTT CTG GAA GAC GGC GTG AAC TAT GCA
ACA GGG AAC CTT CCT GGT TGC TCT TTC TCT ATC TTC CTT CTG GCC
CTG CTC TCT TGC TTG ACC ATC CCC GCT TCC TAA

8. Procédé pour déceler des anticorps dirigés contre un antigène de virus d'hépatite C dans un échantillon expérimental, **caractérisé en ce que**, au cours du procédé, on met en oeuvre des polypeptides suivant la revendication 1.
